# EUROPÄISCHE PATENTSCHRIFT

(11) **EP 2 016 041 B1**
(45) Veröffentlichungstag und Bekanntmachung des Hinweises auf die Patenterteilung: **07.08.2013**
(21) Anmeldenummer: 07724143.8
(22) Anmeldetag: 11.04.2007
(51) Int. Cl.: C07C 68/08, C07C 69/96, A23L 2/44, A23L 3/3463, A23L 3/3517, A23L 3/358, C12H 1/14

(54) **STABILISIERUNG VON DIKOHLENSÄUREDIESTERN MIT PROTONEN-SÄUREN**
STABILIZATION OF DICARBONATE DIESTERS WITH PROTONIC ACIDS
STABILISATION DE DIESTERS D'ACIDE DICARBONIQUE COMPRENANT DES ACIDES PROTONIQUES

(30) Priorität: 22.04.2006 DE 102006018845
(43) Veröffentlichungstag der Anmeldung: 21.01.2009
(73) Patentinhaber: LANXESS Deutschland GmbH, 51369 Leverkusen (DE)
(72) Erfinder: KAHLERT, Steffen, 42799 Leichlingen (DE); KAULEN, Johannes, 51519 Odenthal (DE); VOGL, Erasmus, 51373 Leverkusen (DE)
(86) Internationale Anmeldenummer: PCT/EP2007/003202
(87) Internationale Veröffentlichungsnummer: WO 2007/121859

(56) Entgegenhaltungen:
- WO-A-01/87096
- DE-A1- 3 231 397
- DATABASE WPI Week 197144 Derwent Publications Ltd., London, GB; AN 1971-70699S XP002445031 & JP 46 037810 B (SUMITOMO CHEM CO LTD) 8. November 1971 (1971-11-08) in der Anmeldung erwähnt
- N. N. GREENWOOD: "Chemie der Elemente" 1990, VCH , WEINHEIM , XP002445028 ISBN: 3-527-26169-9 Seite 252 - Seite 255
- KHARITON KHSH ET AL: "HYDROLYSIS IN WATER" ZHURNAL PRIKLADNOI KHIMII JAN 1969, Bd. 42, Nr. 1, Januar 1969 (1969-01), Seiten 236-9, XP008081946
- DATABASE WPI Week 199351 Derwent Publications Ltd., London, GB; AN 1993-408856 XP002445032 & JP 05 306261 A (TOKUYAMA SODA KK) 19. November 1993 (1993-11-19)
- BIZRI J N ET AL: "CITRIC ACID AND ANTIMICROBIALS AFFECT MICROBIOLOGICAL STABILITY AND QUALITY OF TOMATO JUICE" JOURNAL OF FOOD SCIENCE, INSTITUTE OF FOOD TECHNOLOGISTS, CHICAGO, IL, US, Bd. 59, Nr. 1, 1994, Seiten 130-134, XP001079571 ISSN: 0022-1147

## Beschreibung

Die vorliegende Erfindung betrifft die Verwendung von Protonen-Säuren als Stabilisatoren für Dikohlensäurediester, Mischungen enthaltend Dikohlensäurediester und Protonen-Säuren sowie die Verwendung dieser Mischungen zur Konservierung von Lebensmitteln, Getränken und Materialien.

Dikohlensäurediester werden unter anderem zur Konservierung von Lebensmitteln, insbesondere Getränken, als Bestandteile von antimikrobiellen Reagenzien, zur Deaktivierung von Enzymen in Fermentationsprozessen, oder zur Synthese von Feinchemikalien oder Polymeren verwendet. Dikohlensäurediester finden zudem z. B. als Katalysatoren zur Oxidation von Aminen oder zur Synthese, beispielsweise bei der Einführung von Schutzgruppen Verwendung.

Es ist bekannt, dass die Stabilität von Dikohlensäurediestern bei Raumtemperatur und insbesondere bei erhöhter Temperatur relativ gering sein kann. Insbesondere während der Aufreinigung, z. B. einer destillativen Reinigung, oder während einer längeren Lagerung kann es daher zur Zersetzung von Dikohlensäurediestern kommen. Diese Zersetzung kann die Qualität und Reinheit der Dikohlensäurediester verschlechtern. Zudem schreitet die Zersetzung allgemein umso schneller voran, je mehr Verunreinigungen enthalten sind. Eine hohe Reinheit sowie eine Stabilisierung von Dikohlensäurediestern sind daher sehr erstrebenswert.

Aus dem Stand der Technik sind bereits Verfahren zur Verbesserung der thermischen Stabilität von Dikohlensäurediestern bekannt. So wird z. B. vorgeschlagen, Dikohlensäuredialkylester durch den Zusatz von Metallsulfaten zu stabilisieren (vgl. JP-A 48-4016). Nachteilig ist hierbei jedoch, dass diese Metallsulfate mit den Dikohlensäuredialkylestern wenig bis schlecht mischbar sind.

Weiterhin ist bekannt, Dikohlensäuredialkylester durch den Zusatz von Borverbindungen zu stabilisieren (vgl. JP-A 46-37810). Nachteilig ist hierbei jedoch besonders die Toxizität der entsprechenden Borverbindungen. Ein Einsatz in Lebensmitteln kommt für diese Zusätze nicht in Frage.

Weiterhin sind Carbonylverbindungen sowie heteroanaloge Carbonylverbindungen vorgeschlagen worden, als die Lagerstabilität erhöhende Zusatzmittel für Lösungen von Dikohlensäuredialkylestern in gegenüber Dikohlensäuredialkylestern inerten Lösungsmitteln (vgl. DE-A 3 231 397). Allerdings kommen Lösungen von Dikohlensäuredialkylestern in üblichen aprotischen Lösungsmitteln kaum als Zusatz für Lebensmittel in Betracht. Zudem sind stabilisierende Effekte nur mit prozentual relativ großen Mengen an Zusätzen zu erreichen.

Es bestand daher Bedarf an Stabilisatoren, die geeignet sind, Dikohlensäurediester wirkungsvoll gegen thermischen Zerfall zu schützen.

Überraschenderweise wurde nun gefunden, dass Dikohlensäurediester durch Zusatz von verschiedenen Protonen-Säuren gegen thermische und/oder chemische Abbaureaktionen, wie sie z.B. bei der Lagerung oder der destillativen Reinigung auftreten können, stabilisiert werden können.

Gegenstand der vorliegenden Erfindung ist daher die Verwendung von mindestens einer Protonen-Säure aus der Reihe der anorganischen Säuren und der organischen Carbonsäuren und deren Derivate, wobei es sich bei den organischen Carbonsäuren um gesättigte und ein- oder mehrfach ungesättigte aliphatische Monocarbonsäuren und gesättigte und ein- oder mehrfach ungesättigte aliphatische Di- und Polycarbonsäuren und bei deren Derivaten um Hydroxamsäuren, Hydroxycarbonsäuren und um Aldehyd- und Ketosäuren handelt, zur Stabilisierung von Dikohlensäurediestern gegen chemische und/oder thermische Abbaureaktionen, wobei die Protonen-Säure oder deren Mischungen in einer Menge von 0.01 bis 100000 ppm bezogen auf Dikohlensäurediester oder deren Mischungen eingesetzt wird.

Bei den Dikohlensäurediestern handelt es sich bevorzugt um Verbindungen der allgemeinen Formel (1) 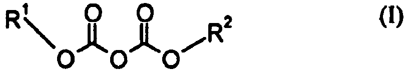 worin
- R¹ und R²: unabhängig voneinander für geradkettiges oder verzweigtes C₁-C₈-Alkyl, Cycloalkyl, C₂-C₈-Alkenyl, C₂-C₈-Alkinyl oder Benzyl stehen,
welches jeweils gegebenenfalls ein- bis mehrfach, gleich oder verschieden substituiert ist durch Halogen; Nitro; Cyano; C₁-C₆-Alkoxy; Dialkylamino; oder für Phenyl stehen welches gegebenenfalls ein bis mehrfach, gleich oder verschieden substituiert ist durch: Halogen; Nitro; Cyano; Alkyl; Halogenalkyl; Alkoxy; Halogenalkoxy; Acyl; Acyloxy; Alkoxycarbonyl; Carboxyl, bevorzugt
- R¹ und R²: unabhängig voneinander für geradkettiges oder verzweigtes C₁-C₈-Alkyl oder C₂-C₈-Alkenyl oder Benzyl stehen,
besonders bevorzugt
- R¹ und R²: unabhängig voneinander für geradkettiges oder verzweigtes C₁-C₅-Alkyl oder C₃-Alkenyl oder Benzyl stehen,
und ganz besonders bevorzugt
- R¹ und R²: unabhängig voneinander für Methyl, Ethyl, Isopropyl, tert.-Butyl, tert.-Amyl, Allyl oder Benzyl stehen.

Bei den erfindungsgemäßen Stabilisatoren handelt es sich um Protonen-Säuren unterschiedlicher Säurestärke.

Als bevorzugte Protonen-Säuren kommen dabei beispielsweise die anorganischen, in der Technik häufig verwendeten, Säuren und deren Derivate sowie organische Carbonsäuren und deren Derivate in Frage.

Besonders bevorzugte anorganische Säuren sind Salzsäure, Schwefelsäure, Schweflige Säure, Salpetersäure, Salpetrige Säure, Hypochlorige Säure, Chlorige Säure, Chlorsäure, Perchlorsäure und ähnliche. Die Säuren werden dabei üblicherweise als wässrige Lösungen eingesetzt.

Als Beispiele für Derivate der anorganischen Säuren (Schwefelsäure und Schweflige Säure) seien besonders die Sulfonsäuren, die Sulfinsäuren und die Sulfamidsäuren genannt. Besonders bevorzugte Derivate aus der Reihe der Sulfonsäuren sind beispielweise Alkylsulfonsäuren, Phenylsulfonsäuren, Methylsulfonsäure, Fluorsulfonsäure und stark saure Ionentauscher wie sie z. B. bekannt sind aus der US-A 6646017. Besonders bevorzugte Derivate aus der Reihe der Sulfamidsäuren sind beispielsweise Cyclohexansulfamidsäuren.

Als organischen Carbonsäuren sind zu nennen: gesättige und ein- oder mehrfach ungesättigte aliphatische Monocarbonsäuren, gesättigte und ein- oder mehrfach ungesättigte aliphatische Di- und Polycarbonsäuren. Als Derivate der organischen Carbonsäuren sind zu nennen: Hydroxycarbonsäuren, Aldehyd- und Ketosäuren und Hydroxamsäuren.

Besonders bevorzugte organische Carbonsäuren sind Ameisensäure, Essigsäure, Propionsäure, Buttersäure, Valeriansäure, Capronsäure, Heptansäure oder längerkettige Säuren wie Fettsäuren. Ebenfalls besonders bevorzugt sind aus der Reihe der mehrfachen Carbonsäuren Oxalsäure, Malonsäure, Bernsteinsäure, Maleinsäure oder Glutarsäure sowie deren Derivate, beispielsweise deren Monoester, wie Methyl- oder Ethylester. Ebenfalls besonders bevorzugt sind aus der Reihe der aliphatischen Mono-, Di- und Polycarbonsäuren Abkömmlinge, welche am Kohlenstoffgerüst zusätzlich weitere OH-Gruppen tragen und deren Derivate, wie beispielsweise Citronensäure, Weinsäure, Äpfelsäure, Milchsäure oder Ascorbinsäure. Hierbei können die mehrfachen Carbonsäuren als partielle Alkyl-Ester vorliegen und zusätzlich können die OH-Gruppen alkyliert oder ebenfalls verestert sein. Auch können weitere organische Reste über die OH-Funktionalitäten an die Carbonsäuren gebunden sein.

Die genannten Carbonsäuren können auch in Form ihrer Salze wie z. B. der Natrium-, Kalium-, Magnesium-, oder Calcium-Salze eingesetzt werden.

Soweit die vorgenannten Carbonsäuren über ein asymmetrisches Kohlenstoff-Atom verfügen, können sowohl die reinen Enantiomeren als auch Enantiomeren- oder Diastereomeren-Gemische eingesetzt werden.

Die Protonen-Säuren können dabei als Reinstoffe oder als wässrige oder alkoholische Lösungen eingesetzt werden. Ebenso können die Verbindungen in Dikohlensäurediestern oder anderen geeigneten Lösungsmitteln vorgelöst werden. Die Protonen-Säuren können auch auf Oberflächen immobilisiert sein.

Zudem können selbstverständlich auch die jeweiligen reaktiven Vorstufen der Säuren, welche in Anwesenheit von Wasser zu den oben genannten Protonen-Säuren hydrolysieren, *in situ* eingesetzt werden. Beispiele hierfür sind etwa Säurechloride oder Säureanhydride.

Die genannten Stabilisatoren werden im allgemeinen in einer Menge von 0.01 bis 100 000 ppm, bevorzugt in einer Menge von 0.1 bis 10 000 ppm, besonders bevorzugt in einer Menge von 0.1 bis 3000 ppm, ganz besonders bevorzugt in einer Menge von 0.1 bis 2000 ppm bezogen auf den Dikohlensäurediester oder deren Gemisch eingesetzt.

Durch die erfindungsgemäße Verwendung gelingt es, Dikohlensäurediester generell gegen thermische und chemische Abbaureaktionen zu stabilisieren. Solche Abbaureaktionen treten z.B. bei der Lagerung auf.

Die erfindungsgemäß stabilisierten Dikohlensäurediester zeichnen sich durch eine verbesserte Lagerstabilität aus. So können die so stabiliserten Dikohlensäurediester über mehrere Monate bei Raumtemperatur gelagert werden, ohne dass eine Zersetzung der Dikohlensäurediester zu beobachten ist.

Weiterer Gegenstand der vorliegenden Erfindung sind Mischungen enthaltend einen oder mehrere Dikohlensäurediester der oben bezeichneten Formel (I) und eine oder mehrere der oben allgemein und bevorzugt beschriebenen Protonen-Säuren im allgemeinen in einer Menge von 0.01 bis 100 000 ppm, bevorzugt in einer Menge von 0.1 bis 10 000 ppm, besonders bevorzugt in einer Menge von 0.1 bis 3000 ppm, ganz besonders bevorzugt in einer Menge von 0.1 bis 2000 ppm, bezogen auf den Dikohlensäurediester oder deren Gemisch.

Ganz besonders bevorzugt sind Mischungen von mindestens einem Dikohlensäurediester der Formel (I), insbesondere von Dimethyldicarbonat und/oder Diethyldicarbonat mit einer oder mehrerer Protonen-Säuren aus der Reihe der bevorzugt und besonders bevorzugt beschriebenen anorganischen Säuren und deren Derivate und der aliphatischen Mono-, Di- und Polycarbonsäuren, welche am Kohlenstoff-Gerüst zusätzlich weitere OH-Gruppen tragen, wie beispielsweise Citronensäure, Weinsäure, Äpfelsäure, Milchsäure oder Ascorbinsäure.

Die erfindungsgemäßen Mischungen können über einen Zeitraum von mehreren Monaten gelagert werden, ohne dass es zu einer Zersetzung der darin enthaltenen Dikohlensäurediester kommt.

Die erfindungsgemäßen Mischungen eignen sich hervorragend zur Konservierung von Lebensmitteln und insbesondere Getränken gegen Befall und/oder Zersetzung durch Mikroorganismen, wie beispielsweise Bakterien, Pilze oder Hefen.

Ebenfalls Gegenstand der vorliegenden Erfindung ist die Verwendung der erfindungsgemäßen Mischungen zur Konservierung von Lebensmitteln und Getränken.

Die erfindungsgemäß stabilisierten Dikohlensäurediester eignen sich zum Beispiel in hervorragender Weise als Kaltentkeimungsmittel für stille oder karbonisierte Getränke wie Soft-Drinks, Vitamin-Getränke, Fruchtsaftgetränke, Teegetränke, alkoholische oder entalkoholisierte Wein-Getränke, Fruchtschorlen oder manche Biere. Üblicherweise werden dazu die Dikohlensäurediester in Mengen zwischen 10 und 250 ppm zeitnah zur Abfüllung den Getränken zugesetzt. Die Zumischung zu den Getränken erfolgt dabei mit speziellen Dosierpumpen. Die Dikohlensäurediester wirken dabei kontrollierend auf eine Reihe von Mikroorganismen wie fermentative Hefen, Schimmel oder fermentative Bakterien. Beispielhaft seien hier etwa genannt Saccharomyces cerevisiae, Mycoderma, Brettanomyces spp, Lactobacillus brevis, Lactobacillus buchneri und viele andere.

Thermische Abbaureaktionen von Dikohlensäurediestern treten des weiteren insbesondere auch bei der Aufreinigung, z. B. bei der Extraktion oder der Destillation von Dikohlensäurediestern auf, wie sie beispielsweise im Rahmen des Herstellungsverfahrens für Dikohlensäurediester durchgeführt wird. Durch die erfindungsgemäße Verwendung von Protonen-Säuren gelingt es, Dikohlensäurediester mit geringeren Verlusten und in größerer Reinheit zu destillieren.

Weiterer Gegenstand der vorliegenden Erfindung ist daher ein Verfahren zur destillativen Reinigung von Dikohlensäurediestern, indem man einen oder mehrere Dikohlensäurediester der oben angegebenen Formel (I) mit einer oder mehreren der oben allgemein bevorzugt und besonders bevorzugt genannten Protonen-Säuren, im allgemeinen in einer Menge von 0.01 bis 100 000 ppm, bevorzugt in einer Menge von 0.1 bis 10 000 ppm, jeweils bezogen auf Dikohlensäurediester oder deren Gemisch, versetzt und anschließend die Mischung bei einem Druck von 5 bis 100 mbar, bevorzugt 10-50 mbar und einer Temperatur zwischen 30 und 120°C, bevorzugt zwischen 40 und 90°C, destilliert. Zur Destillation kommen die in der Technik üblichen Destillationskolonnen in Frage.

Die Ausbeuten an Dikohlensäurediester bei der Destillation liegen üblicherweise > 99%.

Die Stabilisierung von Dikohlensäurediestern kann durch Einstellung einer - verglichen mit hochreinen Dikohlensäurediestern - erhöhten Protonenkonzentration auf einen pH-Wert von unterhalb 6,5, bevorzugt auf einen pH-Wert von 6,0 bis minus 5.0 und besonders bevorzugt von 5,5 bis 0 erfolgen. Dies kann beispielsweise durch die erfindungsgemäße Zugabe von Protonensäuren in Mengen von 0.01 bis 100 000 ppm bewirkt werden.

Die Messung der Protonenkonzentration erfolgt in wässrigen Medien üblicherweise über die sich darin bildenden Oxonium-Ionen. Als übliche Kenngröße wurde daher der pH-Wert definiert. Die Messung des pH-Wertes kann beispielsweise nach geeigneter Probenvorbereitung über Titration mit geeigneten Basen erfolgen. Der Endpunkt der Titration wird dabei üblicherweise durch eine Farbänderung eines Indikatorfarbstoffes indiziert. Die pH-Messung kann jedoch beispielsweise auch auf elektrochemischern Wege erfolgen. Zum Einsatz kommen hier üblicherweise pH-Elektroden, sog. Einstabmessketten.

In organischen Flüssigkeiten kann je nach Feuchtigkeitsgehalt ebenfalls mit pH-Elektroden oft sehr reproduzierbar ein pH-Wert gemessen werden. Eine andere Möglichkeit, im Fall von organischen Flüssigkeiten die Protonenkonzentration zu messen, beinhaltet eine Probenaufbereitung. Beispielsweise kann im Fall von Dikohlensäurediestern die Protonenkonzentration nach Extraktion mit ultrapurem Wasser bestimmt werden. Dabei wird der Dikohlensäurediester als organische Flüssigkeiten mit wenig Wasser versetzt, gut gemischt und die Phasen getrennt. Aus dem pH- Wert der wässrigen Phase kann die ursprünglich vorhandene Säuremenge im Dikohlensäurediester berechnet werden.

Die nachfolgenden Beispiele dienen zur Erläuterung des Gegenstandes der vorliegenden Erfindung ohne diesen jedoch darauf zu beschränken.

### Beispiele

### Beispiel 1

Entsprechend den Angaben in den Tabellen 1 - 4 wurden jeweils definierte Mengen eines bestimmten, hochreinen Dikohlensäurediesters und die jeweils angegebenen Zusätze in einem 10 ml Rundkolben mit Magnetrührer eingewogen. Die jeweils verwendeten, genauen Mengen der Zusätze sind ebenfalls den Tabellen zu entnehmen.

Der Rundkolben wurde mit einem Septum fest verschlossen. In diesem Septum befand sich eine Öffnung, worin ein Teflonschlauch befestigt war, der in eine senkrecht gestellte, mit Silikonöl gefüllte, auf 0.1 ml kalibrierte 50 ml Bürette geleitet wurde. An der Skalierung der Bürette konnte die Menge des sich durch die Zersetzung des Dikohlensäurediesters entwickelnden Kohlendioxids abgelesen werden. Der Kolben wurde zügig in ein, wie in den Tabellen 1 - 4 für den jeweiligen Versuch angegeben, konstant temperiertes Ölbad (gerührt mit 500 rpm) abgesenkt. Die Eintauchtiefe des Kolbens betrug 2,0 cm.

Nach der jeweils angegebenen Zeit, in der Regel nach 1, 2, 5, 10 und 15 Minuten, wurde das Gasvolumen abgelesen. Das Gasvolumen ist ein Maß für den Grad der Zersetzung der Dikohlensäurediester in CO₂. Es spiegelt damit umgekehrt den Grad der Stabilisierung durch die geprüften Zusätze wieder.

In den meisten Fällen wurden die Versuche wiederholt, um Reproduzierbarkeit sicherzustellen. Aussagekräftige Reproduzierbarkeit war jeweils gegeben.

Die Ergebnisse sind den angehängten Tabellen zu entnehmen. Hochreiner Dikohlensäurediester setzte in der beobachteten Zeit wenig Kohlendioxid frei, doch schon in Kontakt mit geringen Mengen von Silicagel, Braunstein oder auch nur rauhen Oberflächen, wie zerkratztem Glas, wurde die Zersetzung drastisch beschleunigt. Geringe Mengen der Stabilisatoren reichten aus um die Zersetzung effektiv zu verringern.

Je weniger gasförmige Zersetzungsprodukte Dikohlensäurediester unter Temperaturbelastung abgeben, desto günstiger wird eine Destillation unter Vakuum verlaufen.

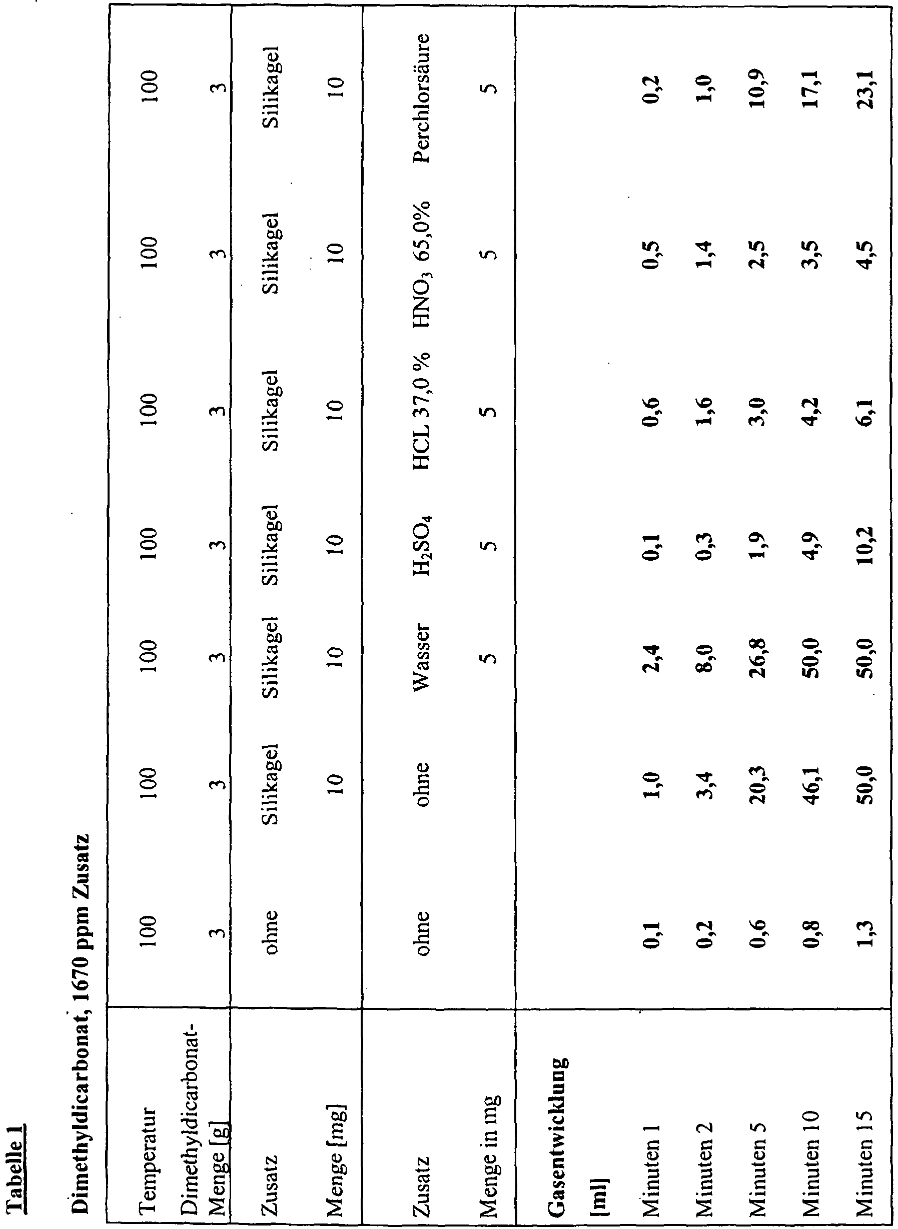

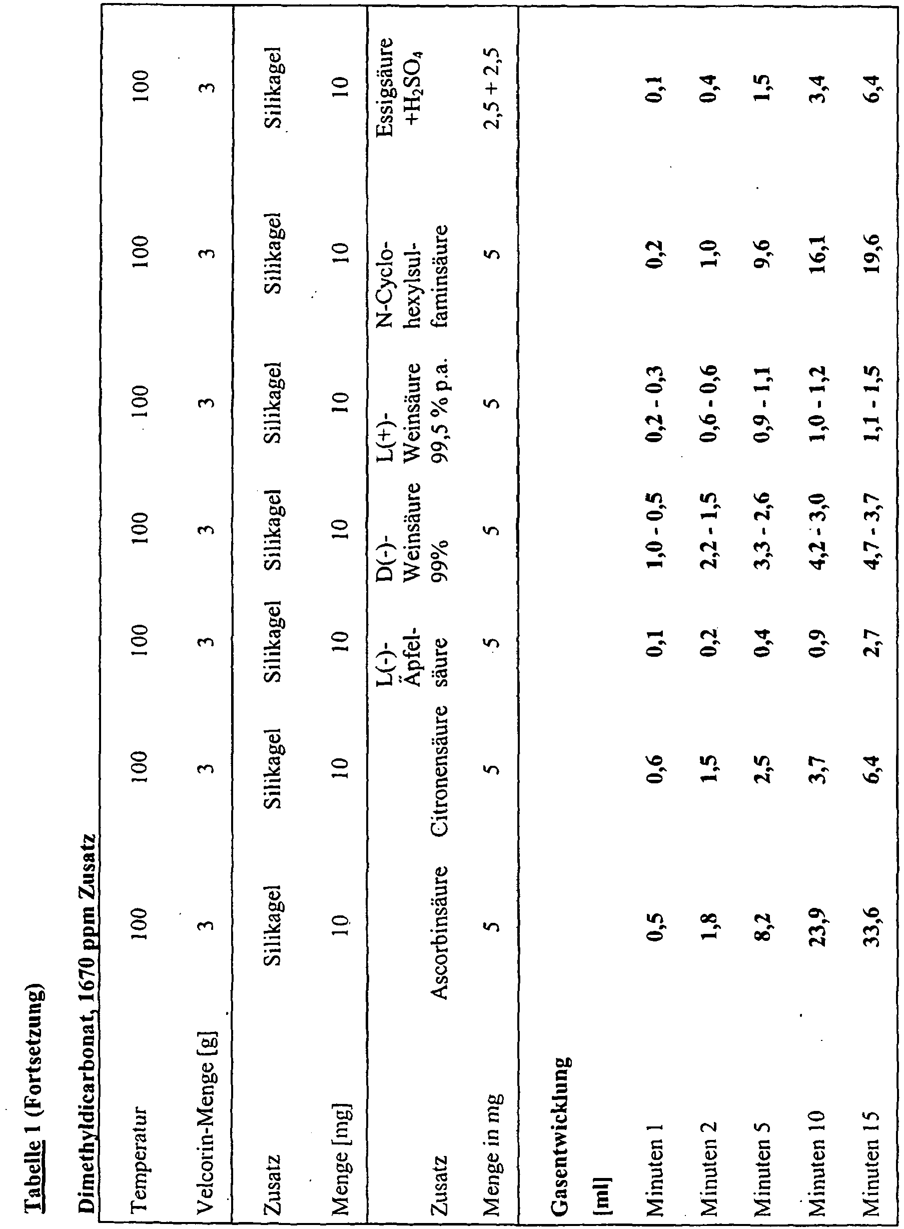

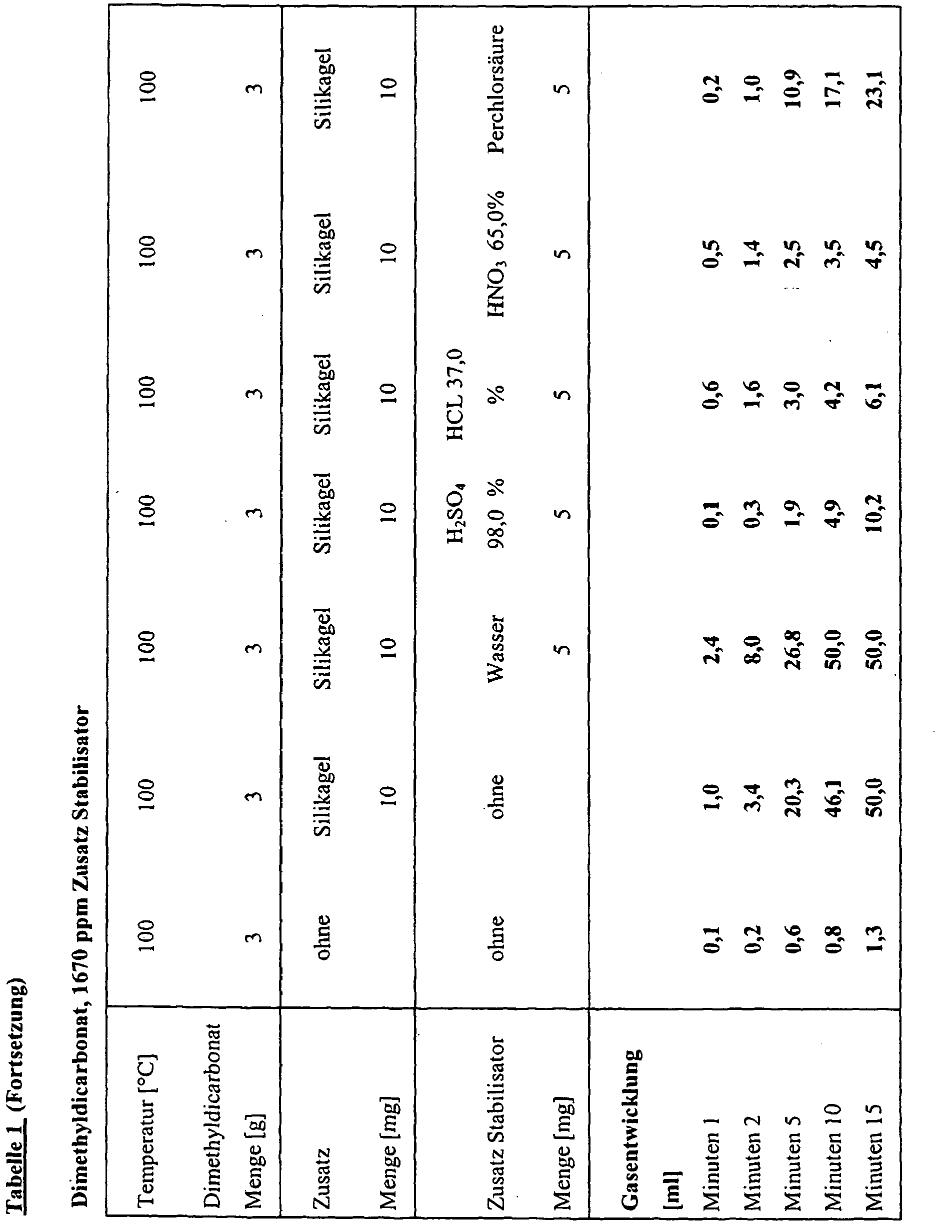

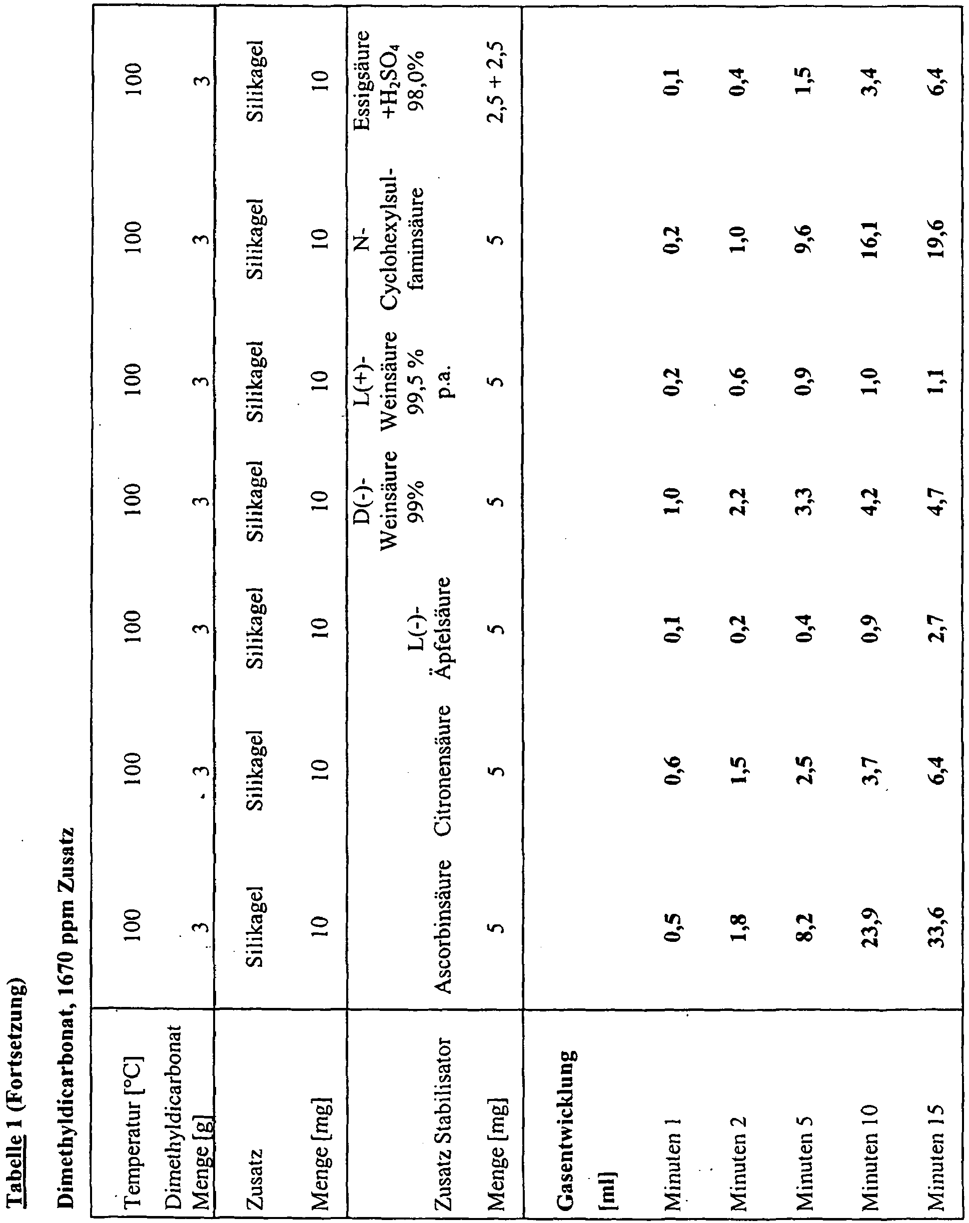

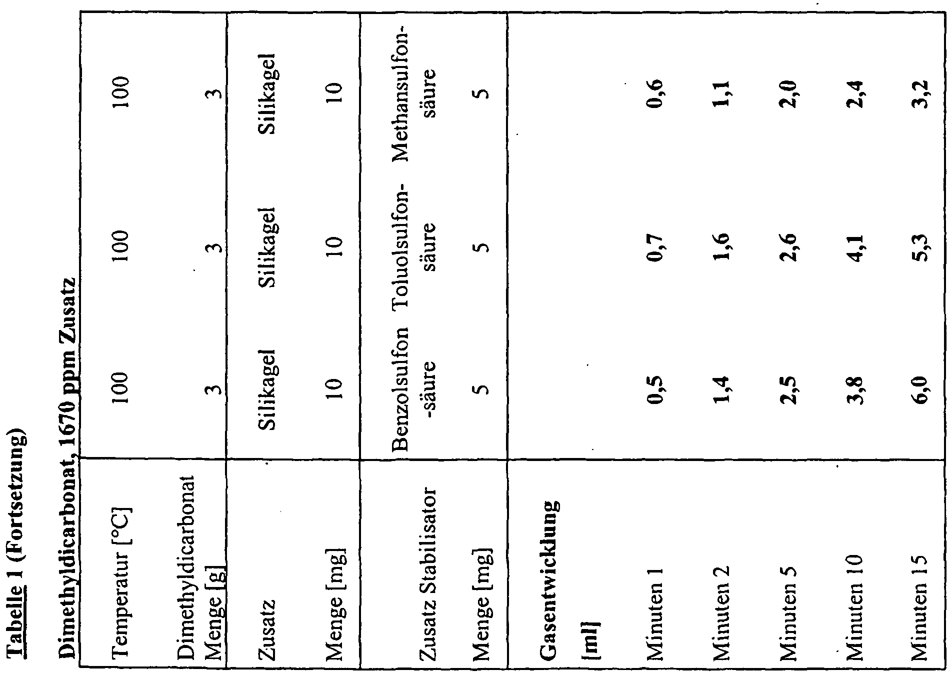

**Tabelle 2**

| **Dimethyldicarbonat 1670 ppm Zusatz Stabilisator** | | | | | | | |
|---|---|---|---|---|---|---|---|
| Temperatur [°C] | 100 | 100 | 100 | 100 | 100 | 100 | 100 |
| Dimethyldicarbonat Menge [g] | 3 | 3 | 3 | 3 | 3 | 3 | 3 |
| Zusatz | Braunstein | Braunstein | Braunstein | Braunstein | Braunstein | Braunstein | Braunstein |
| Menge [mg] | 10 | 10 | 10 | 10 | 10 | 10 | 10 |
| Zusatz Stabilisator | ohne | HCl 37,0 % | HNO₃ 65,0% | Citronensäure | L(-)-Äpfelsäure | L(+)-Weinsäure 99,5 % p.a. | Wasser |
| Menge [mg] | | 5 | 5 | 5 | 5 | 5 | 5 |
| **Gasentwicklung [ml]** | | | | | | | |
| Minuten 1 | **3,8** | **0,9** | **0,9** | **2,5** | **0,5** | **2,9** | **7,1** |
| Minuten 2 | **9,3** | **2,0** | **2,8** | **5,9** | **1,2** | **6,2** | **26,4** |
| Minuten 5 | **21,7** | **6,9** | **7,4** | **8,8** | **2,7** | **9,0** | **35,4** |
| Minuten 10 | **31,1** | **9,6** | **13,6** | **11,7** | **3,9** | **10,5** | **46,1** |
| Minuten 15 | **41,5** | **12,2** | **19,6** | **15,9** | **5,3** | **11,2** | **50,0** |

**Tabelle 3**

| **Dimethyldicarbonat,1670 ppm Zusatz Stabilisator** | | | |
|---|---|---|---|
| Temperatur [°C] | 100 | 100 | 100 |
| Dimethyldicarbonat Menge [g] | 3 | 3 | 3 |
| Zusatz | ohne | Oberfläche des Kolbens innen stark zerkratzt | Oberfläche des Kolbens innen stark zerkratzt |
| Zusatz Stabilisator | ohne | ohne | L(+)-Weinsäure |
| Menge [mg] | | | 5 |
| Gasentwicklung [ml] | | | |
| Minuten 1 | **0,1** | **0,7** | **0,6** |
| Minuten 2 | **0,2** | **1,3** | **1,4** |
| Minuten 5 | **0,5** | **2,2** | **2,1** |
| Minuten 10 | **1,3** | **3,8** | **2,7** |
| Minuten 15 | **2,7** | **5,7** | **3,0** |

**Tabelle 4**

| **Dimethyldicarbonat, <1000 ppm Zusatz Stabilisator** | | | | | |
|---|---|---|---|---|---|
| Temperatur [°C] | 100 | 100 | 100 | 100 | 100 |
| Dimethyldicarbonat Menge [g] | 3 | 3 | 3 | 3 | 3 |
| Zusatz | Silikagel | Silikagel | Silikagel | Silikagel | Silikagel |
| Menge [mg] | 10 | 10 | 10 | 10 | 10 |
| | | 10 ppm | 100 ppm | 100 ppm | 100 ppm |
| | 100 ppm | H₂SO₄ | H₂SO₄ | L(+)- | HNO₃ |
| Zusatz Stabilisator | Äpfelsäure | 98,0% | 98,0% | Weinsäure | 65,0% |

| **Gasentwicklung [ml]** | | | | | |
|---|---|---|---|---|---|
| Minuten 1 | **0,2** | **0,7** | **0,9** | **0,4** | **0,2** |
| Minuten 2 | **1,4** | **1,9** | **1,5** | **1,7** | **0,6** |
| Minuten 5 | **14,3** | **11,2** | **2,7** | **16,1** | **2,5** |
| Minuten 10 | **40,4** | **33,5** | **4,2** | **38,7** | **34,4** |
| Minuten 15 | | | **15,5** | | |

### Beispiel 2

Der Zusammenhang zwischen Stabilisierung und dem direkt mit einer pH-Elektrode betimmten pH-Wert, wurde experimentell gemessen. Hochreiner Dikohlensäuredimethylester wurde zunächst mit unterschiedlichen Mengen Säure oder Base versetzt. Der sich daraufhin einstellende, pH-Wert wurde direkt mit einer pH-Elektrode (Einstabmesskette) der Firma Mettler Toledo, Modell Inlab 1010, gemessen. Die Elektrode wurde vorher in Pufferlösungen geeicht. Daraufhin wurde die Zersetzlichkeit in Abhängigkeit vom pH-Wert experimentell, analog dem Vorgehen in Beispiel 1, bestimmt. Die jeweiligen Werte für die Kohlendioxidentwicklung sind der Tabelle zu entnehmen. Man erkennt einen klaren Zusammenhang zwischen Protonengehalt und Stabilität. Ebenso konnte die im Dikohlensäurediester anwesende anorganische Säuremenge nach Extraktion mit ultrapurem Wasser bestimmt werden. Dazu wurde der Dikohlensäuredimethylester mit wenig hochreinem Wasser extrahiert und vor oder nach Phasentrennung der pH-Wert mit der Glaselektrode in diesem Wasser gemessen. Danach wurde der pH-Wert auf das ursprüngliche Dicarbonat-Volumen umgerechnet. Dieses Vorgehen ergab identische Werte und weniger Drift der pH-Elektrode. Die Lösungen wurden bei den pH-Bestimmungen mit Argon gespült um Beeinflussungen des pH-Wertes durch Kohlendioxid zu verhindern.

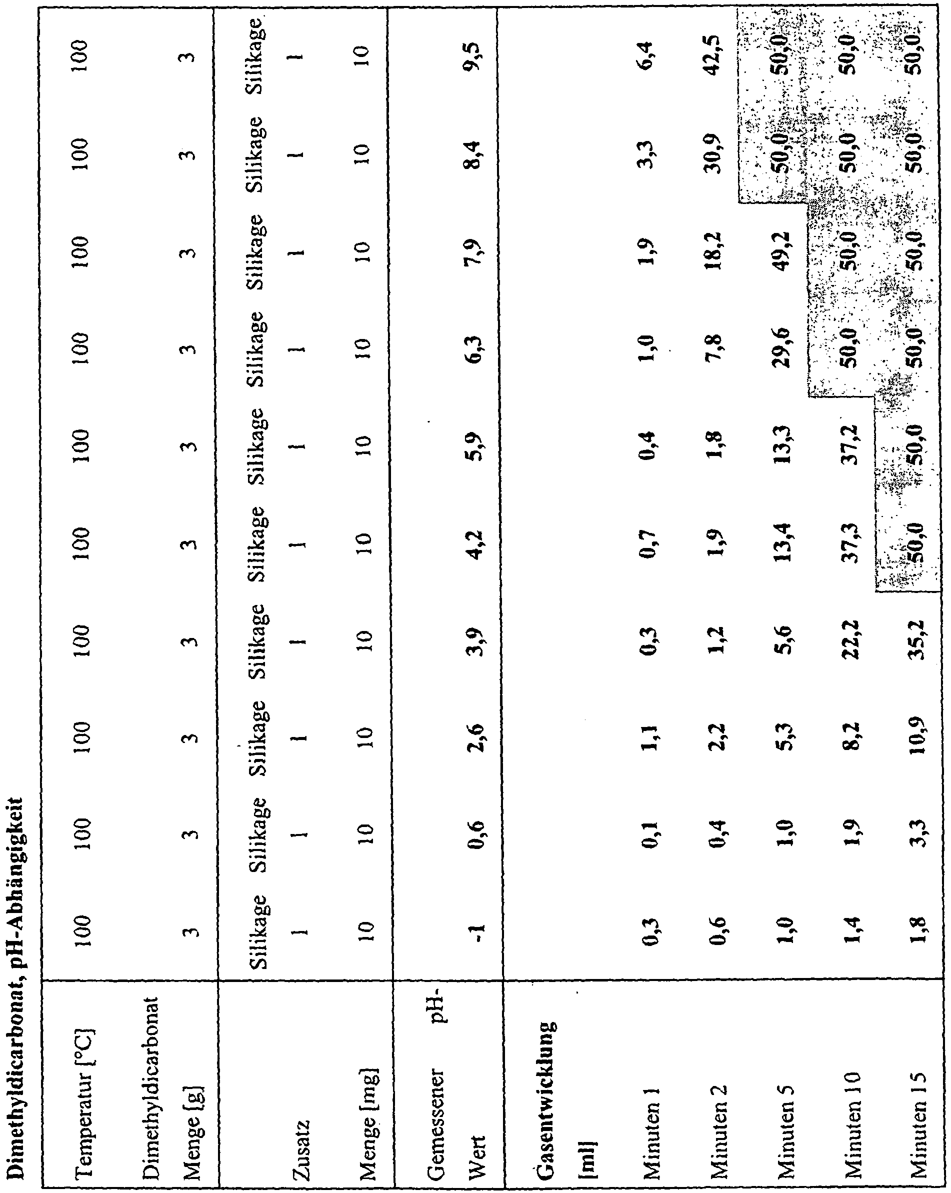

## Patentansprüche

1. Verwendung von mindestens einer Protonen-Säure aus der Reihe der anorganischen Säuren und der organischen Carbonsäuren und deren Derivate, wobei es sich bei den organischen Carbonsäuren um gesättigte und ein- oder mehrfach ungesättigte aliphatische Monocarbonsäuren und gesättigte und ein- oder mehrfach ungesättigte aliphatische Di- und Polycarbonsäuren und bei deren Derivaten, um Hydroxamsäuren, Hydroxycarbonsäuren, Aldehyd- und Ketosäuren handelt, zur Stabilisierung von Dikohlensäurediestern gegen chemische und thermische Abbaureaktionen, **dadurch gekennzeichnet, dass** die Protonen-Säure oder deren Mischungen in einer Menge von 0.01 bis 100 000 ppm bezogen auf Dikohlensäurediester oder deren Mischungen eingesetzt wird.

2. Verwendung gemäß Anspruch 1, **dadurch gekennzeichnet, dass** es sich bei den anorganische Säuren um Salzsäure, Schwefelsäure, Schweflige Säure, Salpetersäure, Salpetrige Säure, Hypochlorige Säure, Chlorige Säure, Chlorsäure, Perchlorsäure handelt.

3. Verwendung gemäß wenigsten einem der Ansprüche 1 und 2, **dadurch gekennzeichnet dass** es sich bei den Derivaten der anorganischen Säuren um Alkylsulfonsäuren, Phenylsulfonsäuren, Methylsulfonsäure Fluorsulfonsäure und stark saure Ionentauscher handelt.

4. Verwendung gemäß Anspruch 1, **dadurch gekennzeichnet, dass** es sich bei den organischen Carbonsäuren um Ameisensäure, Essigsäure, Propionsäure, Buttersäure, Valeriansäure, Capronsäure, Heptansäure, längerkettige gesättigte und ungesättigte Fettsäuren, Oxalsäure, Malonsäure, Bernsteinsäure, Maleinsäure, Glutarsäure, Citronensäure, Weinsäure, Äpfelsäure, Milchsäure oder Ascorbinsäure oder deren Derivate handelt.

5. Verwendung gemäß wenigstens einem der Ansprüche 1 bis 4, **dadurch gekennzeichnet, dass** es sich bei den Dikohlensäurediestern um Verbindungen der allgemeinen Formel 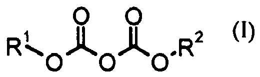 worin
R¹ und R² unabhängig voneinander für geradkettiges oder verzweigtes C₁-C₈-Alkyl, Cycloalkyl, C₂-C₈-Alkenyl, C₂-C₈-Alkinyl oder Benzyl stehen, welches jeweils gegebenenfalls ein- bis mehrfach, gleich oder verschieden substituiert ist durch Halogen; Nitro; Cyano; C₁-C₆-Alkoxy; Dialkylamino; oder für Phenyl, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl; stehen,
handelt.

6. Verwendung gemäß wenigstens einem der Ansprüche 1 bis 5, **dadurch gekennzeichnet, dass** es sich bei den Dikohlensäurediestern um Dikohlensäuredimethylester oder Dikohlensäurediethylester handelt.

7. Verwendung gemäß wenigstens einem der Ansprüche 1 bis 6, **dadurch gekennzeichnet, dass** es sich um die Stabilisierung gegen Abbaureaktionen bei der Aufarbeitung, Extraktion, Destillation oder Lagerung handelt.

8. Mischungen enthaltend einen oder mehrere Dikohlensäurediester der allgemeinen Formel 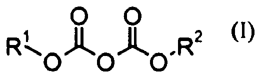 worin
R¹ und R² unabhängig voneinander für geradkettiges oder verzweigtes C₁-C₈-Alkyl, Cycloalkyl, C₂-C₈-Alkenyl, C₂-C₈-Alkinyl oder Benzyl stehen,
welches jeweils gegebenenfalls ein- bis mehrfach, gleich oder verschieden substituiert ist durch Halogen; Nitro; Cyano; C₁-C₆-Alkoxy; Dialkylamino; oder für Phenyl, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl, stehen,
und eine oder mehrere Protonen-Säuren aus der Reihe der anorganischen Säuren und der organischen Carbonsäuren und deren Derivate, wobei es sich bei den organischen Carbonsäuren um gesättigte und ein- oder mehrfach ungesättigte aliphatische Monocarbonsäuren und gesättigte und ein- oder mehrfach ungesättigte aliphatische Di- und Polycarbonsäuren und bei deren Derivaten, um Hydroxamsäuren, Hydroxycarbonsäuren, Aldehyd- und Ketosäuren handelt, **dadurch gekennzeichnet, dass** die Protonen-Säure oder deren Mischungen in einer Menge von 0.01 bis 100 000 ppm bezogen auf Dikohlensäurediester oder deren Mischungen eingesetzt wird.

9. Mischung gemäß Anspruch 8, **dadurch gekennzeichnet, dass** es sich bei den anorganische Säuren und deren Derivaten um Salzsäure, Schwefelsäure, Schweflige Säure, Salpetersäure, Salpetrige Säure, Hypochlorige Säure, Chlorige Säure, Chlorsäure Perchlorsäure, Sulfonsäuren oder stark saure Ionentauscher handelt.

10. Mischung gemäß wenigstens einem der Ansprüche 8 oder 9, **dadurch gekennzeichnet, dass** sie mindestens eine Verbindung aus der Reihe Dimethyldicarbonat und Diethyldicarbonat und mindestens eine Protonen-Säure aus der Reihe Salzsäure, Schwefelsäure, Schweflige Säure, Salpetersäure, Salpetrige Säure, Hypochlorige Säure, Chlorige Säure, Chlorsäure, Perchlorsäure, Ameisensäure, Essigsäure, Propionsäure, Buttersäure, Valeriansäure, Capronsäure, Heptansäure, längerkettige gesättigte und ungesättigte Fettsäuren, Oxalsäure, Malonsäure, Bernsteinsäure, Maleinsäure, Glutarsäure, Citronensäure, Weinsäure, Äpfelsäure, Milchsäure oder Ascorbinsäure oder deren Derivate handelt.

11. Verwendung einer Mischung gemäß wenigstens einem der Ansprüche 8 bis 10 zur Konservierung von Lebensmitteln, Getränken und Materialien.

12. Verfahren zur destillativen Reinigung von Dikohlensäuredialkylestern **dadurch gekennzeichnet, dass** man einen oder mehrere Dikohlensäuredialkylester der allgemeinen Formel 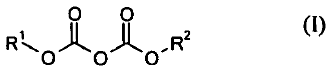 worin
R¹ und R² unabhängig voneinander für geradkettiges oder verzweigtes C₁-C₈-Alkyl, Cycloalkyl, C₂-C₈-Alkenyl, C₂-C₈-Alkinyl oder Benzyl stehen,
welches jeweils gegebenenfalls ein- bis mehrfach, gleich oder verschieden substituiert ist durch Halogen; Nitro; Cyano; C₁-C₆-Alkoxy; Dialkylamino; oder für Phenyl, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl; stehen,
mit einer oder mehreren Protonen-Säuren aus der Reihe der anorganischen Säuren und der organischen Carbonsäuren und deren Derivate in einer Menge von 0.01 bis 100 000 ppm bezogen auf Dikohlensäurediester oder deren Mischungen versetzt und anschließend die Mischung destilliert wird, **dadurch gekennzeichnet, dass** es sich bei den anorganischen Säuren und der organischen Carbonsäuren und deren Derivate um gesättigte und ein- oder mehrfach ungesättigte aliphatische Monocarbonsäuren und gesättigte und ein- oder mehrfach ungesättigte aliphatische Di- und Polycarbonsäuren und bei deren Derivaten, um Hydroxamsäuren, Hydroxycarbonsäuren, Aldehyd- und Ketosäuren handelt..

13. Verfahren gemäß Anspruch 12, **dadurch gekennzeichnet, dass** es sich es sich bei den Protonen-Säuren um solche aus der Reihe Salzsäure, Schwefelsäure, Schweflige Säure, Salpetersäure, Salpetrige Säure, Hypochlorige Säure, Chlorige Säure, Chlorsäure, Perchlorsäure, Sulfonsäuren, Ameisensäure, Essigsäure, Propionsäure, Buttersäure, Valeriansäure, Capronsäure, Heptansäure, längerkettige gesättigte und ungesättigte Fettsäuren, Oxalsäure, Malonsäure, Bernsteinsäure, Maleinsäure, Glutarsäure , Citronensäure, Weinsäure, Äpfelsäure, Milchsäure oder Ascorbinsäure oder deren Derivate handelt.

14. Verfahren gemäß wenigstens einem der Ansprüche 12 oder 13, **dadurch gekennzeichnet, dass** man bei einem Druck von 5 bis 100 mbar und einer Temperatur zwischen 30 und 120°C destilliert.

## Claims

1. Use of at least one protonic acid from the series of inorganic acids and organic carboxylic acids and derivatives thereof, the organic carboxylic acids being saturated and monounsaturated or polyunsaturated aliphatic monocarboxylic acids and saturated and monounsaturated or polyunsaturated aliphatic dicarboxylic and polycarboxylic acids and derivatives thereof being hydroxamic acids, hydroxycarboxylic acids, aldehyde and keto acids, for stabilization of diesters of dicarbonic acid against chemical and thermal breakdown reactions, **characterized in that** the protonic acid or mixtures thereof is used in an amount of 0.01 to 100 000 ppm, based on diesters of dicarbonic acid or mixtures thereof.

2. Use according to Claim 1, **characterized in that** the inorganic acids are hydrochloric acid, sulphuric acid, sulphurous acid, nitric acid, nitrous acid, hypochlorous acid, chlorous acid, chloric acid, perchloric acid.

3. Use according to at least one of Claims 1 and 2, **characterized in that** the derivatives of the inorganic acids are alkylsulphonic acids, phenylsulphonic acids, methylsulphonic acid, fluorosulphonic acid and strongly acidic ion exchangers.

4. Use according to Claim 1, **characterized in that** the organic carboxylic acids are formic acid, acetic acid, propionic acid, butyric acid, valeric acid, caproic acid, heptanoic acid, longer-chain saturated and unsaturated fatty acids, oxalic acid, malonic acid, succinic acid, maleic acid, glutaric acid, citric acid, tartaric acid, malic acid, lactic acid or ascorbic acid or derivatives thereof.

5. Use according to at least one of Claims 1 to 4, **characterized in that** the diesters of dicarbonic acid are compounds of the general formula 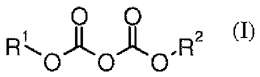 where
R¹ and R² independently of one another are straight-chain or branched C₁-C₈-alkyl, cycloalkyl, C₂-C₈-alkenyl, C₂-C₈-alkynyl or benzyl, each of which is optionally monosubstituted to polysubstituted, identically or differently by halogen; nitro; cyano; C₁-C₆-alkoxy; dialkylamino; or are phenyl which is optionally monosubstituted to polysubstituted, identically or differently by halogen; nitro; cyano; alkyl; haloalkyl; alkoxy; haloalkoxy; acyl; acyloxy; alkoxycarbonyl; carboxyl.

6. Use according to at least one of Claims 1 to 5, **characterized in that** the diesters of dicarbonic acid are dimethyl dicarbonate or diethyl dicarbonate.

7. Use according to at least one of Claims 1 to 6, **characterized in that** the stabilization against breakdown reactions is during workup, extraction, distillation or storage.

8. Mixtures containing one or more diesters of dicarbonic acid of the general formula 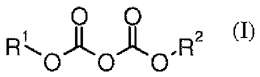 where
R¹ and R² independently of one another are straight-chain or branched C₁-C₈-alkyl, cycloalkyl, C₂-C₈-alkenyl, C₂-C₈-alkynyl or benzyl,
each of which is optionally monosubstituted to polysubstituted, identically or differently by halogen; nitro; cyano; C₁-C₆-alkoxy; dialkylamino; or are phenyl which is optionally monosubstituted to polysubstituted, identically or differently by halogen; nitro; cyano; alkyl; haloalkyl; alkoxy; haloalkoxy; acyl; acyloxy; alkoxycarbonyl; carboxyl,
and one or more protonic acids from the series of inorganic acids and organic carboxylic acids and derivatives thereof, the organic carboxylic acids being saturated and monounsaturated or polyunsaturated aliphatic monocarboxylic acids and saturated and monounsaturated or polyunsaturated aliphatic dicarboxylic and polycarboxylic acids and derivatives thereof being hydroxamic acids, hydroxycarboxylic acids, aldehyde and keto acids, **characterized in that** the protonic acid or mixtures thereof is used in an amount of 0.01 to 100 000 ppm, based on diesters of dicarbonic or mixtures thereof.

9. Mixture according to Claim 8, **characterized in that** the inorganic acids and derivatives thereof are hydrochloric acid, sulphuric acid, sulphurous acid, nitric acid, nitrous acid, hypochlorous acid, chlorous acid, chloric acid, perchloric acid, sulphonic acids or strongly acidic ion exchangers.

10. Mixture according to at least one of Claims 8 and 9, **characterized in that** it is at least one compound from the series dimethyl dicarbonate and diethyl dicarbonate and at least one protonic acid from the series hydrochloric acid, sulphuric acid, sulphurous acid, nitric acid, nitrous acid, hypochlorous acid, chlorous acid, chloric acid, perchloric acid, formic acid, acetic acid, propionic acid, butyric acid, valeric acid, caproic acid, heptanoic acid, longer-chain saturated and unsaturated fatty acids, oxalic acid, malonic acid, succinic acid, maleic acid, glutaric acid, citric acid, tartaric acid, malic acid, lactic acid or ascorbic acid or derivatives thereof.

11. Use of a mixture according to at least one of Claims 8 to 10 for preservation of foods, drinks and materials.

12. Method for the purification by distillation of dialkyl dicarbonates, **characterized in that** one or more dialkyl dicarbonates of the general formula 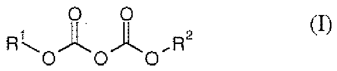 where
R¹ and R² independently of one another are straight-chain or branched C₁-C₈-alkyl, cycloalkyl, C₂-C₈-alkenyl, C₂-C₈-alkynyl or benzyl,
each of which is optionally monosubstituted to polysubstituted, identically or differently by halogen; nitro; cyano; C₁-C₆-alkoxy; dialkylamino; or are phenyl which is optionally monosubstituted to polysubstituted, identically or differently by halogen; nitro; cyano; alkyl; haloalkyl; alkoxy; haloalkoxy; acyl; acyloxy; alkoxycarbonyl; carboxyl,
is admixed with one or more protonic acids from the series of inorganic acids and organic carboxylic acids and derivatives thereof in an amount of 0.01 to 100 000 ppm, based on diesters of dicarbonic acid or mixtures thereof and the mixture is subsequently distilled, **characterized in that** the inorganic acids and organic carboxylic acids and derivatives thereof are saturated and monounsaturated or polyunsaturated aliphatic monocarboxylic acids and saturated and monounsaturated or polyunsaturated aliphatic dicarboxylic and polycarboxylic acids and derivatives thereof are hydroxamic acids, hydroxycarboxylic acids, aldehyde and keto acids.

13. Method according to Claim 12, **characterized in that** the protonic acids are those of the series hydrochloric acid, sulphuric acid, sulphurous acid, nitric acid, nitrous acid, hypochlorous acid, chlorous acid, chloric acid, perchloric acid, sulphonic acids, formic acid, acetic acid, propionic acid, butyric acid, valeric acid, caproic acid, heptanoic acid, longer-chain saturated and unsaturated fatty acids, oxalic acid, malonic acid, succinic acid, maleic acid, glutaric acid, citric acid, tartaric acid, malic acid, lactic acid or ascorbic acid or derivatives thereof.

14. Method according to at least one of Claims 12 and 13, **characterized in that** distillation is carried out at a pressure of 5 to 100 mbar and a temperature between 30 and 120°C.

## Revendications

1. Utilisation d'au moins un acide protonique de la série des acides inorganiques et des acides carboxyliques organiques et leurs dérivés, où il s'agit, pour les acides carboxyliques organiques, d'acides monocarboxyliques aliphatiques saturés et monoinsaturés ou polyinsaturés et d'acides dicarboxyliques et polycarboxyliques aliphatiques saturés et monoinsaturés ou polyinsaturés et, pour leurs dérivés, d'acides hydroxamiques, d'acides hydroxycarboxyliques, d'aldéhyde-acides et de cétoacides, pour la stabilisation de diesters de l'acide dicarbonique contre des réactions de dégradation chimiques et thermiques, **caractérisée en ce que** l'acide protonique ou ses mélanges est/sont utilisé(s) en une quantité de 0,01 à 100 000 ppm par rapport aux diesters de l'acide dicarbonique ou à ses mélanges.

2. Utilisation selon la revendication 1, **caractérisée en ce qu'**il s'agit, pour les acides inorganiques, d'acide chlorhydrique, d'acide sulfurique, d'acide sulfureux, d'acide nitrique, d'acide nitreux, d'acide hypochlorique, d'acide chloreux, d'acide chlorique, d'acide perchlorique.

3. Utilisation selon au moins l'une quelconque des revendications 1 et 2, **caractérisée en ce qu'**il s'agit, pour les dérivés des acides inorganiques, d'acides alkylsulfoniques, d'acides phénylsulfoniques, d'acide méthylsulfonique, d'acide fluorosulfonique et d'échangeurs ioniques acides forts.

4. Utilisation selon la revendication 1, **caractérisée en ce qu'**il s'agit, pour les acides carboxyliques organiques, d'acide formique, d'acide acétique, d'acide propionique, d'acide butyrique, d'acide valérianique, d'acide caproïque, d'acide heptanoïque, d'acides gras à plus longue chaîne, saturés et insaturés, d'acide oxalique, d'acide malonique, d'acide succinique, d'acide maléique, d'acide glutarique, d'acide citrique, d'acide tartrique, d'acide malique, d'acide lactique ou d'acide ascorbique ou de leurs dérivés.

5. Utilisation selon au moins l'une quelconque des revendications 1 à 4, **caractérisée en ce qu'**il s'agit, pour les diesters de l'acide dicarbonique, de composés de formule générale 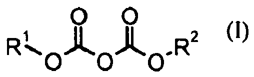 où
R¹ et R² représentent, indépendamment l'un de l'autre, C₁-C₈-alkyle linéaire ou ramifié, cycloalkyle, C₂-C₈-alcényle, C₂-C₈-alcynyle ou benzyle, qui est à chaque fois le cas échéant monosubstitué à polysubstitué, de manière identique ou différente par halogène ; nitro ; cyano ; C₁-C₆-alcoxy ; dialkylamino ; ou représentent phényle, qui est le cas échéant monosubstitué à polysubstitué, de manière identique ou différente, par halogène ; nitro ; cyano ; alkyle ; halogénoalkyle ; alcoxy ; halogénoalcoxy ; acyle ; acyloxy ; alcoxycarbonyle ; carboxyle.

6. Utilisation selon au moins l'une quelconque des revendications 1 à 5, **caractérisée en ce qu'**il s'agit, pour les diesters de l'acide dicarbonique, de l'ester diméthylique de l'acide dicarbonique ou de l'ester diéthylique de l'acide dicarbonique.

7. Utilisation selon au moins l'une quelconque des revendications 1 à 6, **caractérisée en ce qu'**il s'agit de la stabilisation contre des réactions de dégradation lors du traitement, de l'extraction, de la distillation ou de l'entreposage.

8. Mélanges contenant un ou plusieurs diesters de l'acide dicarbonique de formule générale 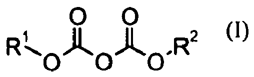 où
R¹ et R² représentent, indépendamment l'un de l'autre, C₁-C₈-alkyle linéaire ou ramifié, cycloalkyle, C₂-C₈-alcényle, C₂-C₈-alcynyle ou benzyle, qui est à chaque fois le cas échéant monosubstitué à polysubstitué, de manière identique ou différente par halogène ; nitro ; cyano ; C₁-C₆-alcoxy ; dialkylamino ; ou représentent phényle, qui est le cas échéant monosubstitué à polysubstitué, de manière identique ou différente, par halogène ; nitro ; cyano ; alkyle ; halogénoalkyle ; alcoxy ; halogénoalcoxy ; acyle ; acyloxy ; alcoxycarbonyle ; carboxyle,
et un ou plusieurs acides protoniques de la série des acides inorganiques et des acides carboxyliques organiques et leurs dérivés, où il s'agit, pour les acides carboxyliques organiques, d'acides monocarboxyliques aliphatiques saturés et monoinsaturés ou polyinsaturés et d'acides dicarboxyliques ou polycarboxyliques aliphatiques saturés et monoinsaturés ou polyinsaturés et, pour leurs dérivés, d'acides hydroxamiques, d'acides hydroxycarboxyliques, d'aldéhyde-acides et de cétoacides, **caractérisés en ce que** l'acide protonique ou ses mélanges est/sont utilisé(s) en une quantité de 0,01 à 100 000 ppm par rapport au diester de l'acide dicarbonique.

9. Mélange selon la revendication 8, **caractérisé en ce qu'**il s'agit, pour les acides inorganiques et leurs dérivés, d'acide chlorhydrique, d'acide sulfurique, d'acide sulfureux, d'acide nitrique, d'acide nitreux, d'acide hypochlorique, d'acide chloreux, d'acide chlorique, d'acide perchlorique, d'acides sulfoniques ou d'échangeurs ioniques acides forts.

10. Mélange selon au moins l'une quelconque des revendications 8 ou 9, **caractérisé en ce qu'**il contient au moins un composé de la série diméthyldicarbonate et diéthyldicarbonate et au moins un acide protonique de la série acide chlorhydrique, acide sulfurique, acide sulfureux, acide nitrique, acide nitreux, acide hypochloreux, acide chloreux, acide chlorique, acide perchlorique, acide formique, acide acétique, acide propionique, acide butyrique, acide valérianique, acide caproïque, acide heptanoïque, acides gras à plus longue chaîne, saturés et insaturés, acide oxalique, acide malonique, acide succinique, acide maléique, acide glutarique, acide citrique, acide tartrique, acide malique, acide lactique ou acide ascorbique ou leurs dérivés.

11. Utilisation d'un mélange selon au moins l'une quelconque des revendications 8 à 10 pour la conservation d'aliments, de boissons et de matériaux.

12. Procédé pour la purification par distillation d'esters dialkyliques d'acide dicarbonique, **caractérisé en ce qu'**on mélange un ou plusieurs esters dialkyliques d'acide dicarbonique de formule générale 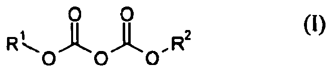 où
R¹ et R² représentent, indépendamment l'un de l'autre, C₁-C₈-alkyle linéaire ou ramifié, cycloalkyle, C₂-C₈-alcényle, C₂-C₈-alcynyle ou benzyle, qui est à chaque fois le cas échéant monosubstitué à polysubstitué, de manière identique ou différente par halogène ; nitro ; cyano ; C₁-C₆-alcoxy ; dialkylamino ; ou représentent phényle, qui est le cas échéant monosubstitué à polysubstitué, de manière identique ou différente, par halogène ; nitro ; cyano ; alkyle ; halogénoalkyle ; alcoxy ; halogénoalcoxy ; acyle ; acyloxy ; alcoxycarbonyle ; carboxyle,
avec un ou plusieurs acides protoniques de la série des acides inorganiques et des acides carboxyliques organiques et leurs dérivés en une quantité de 0,01 à 100 000 ppm par rapport au diester de l'acide dicarbonique ou leurs mélanges et le mélange est ensuite distillé, **caractérisé en ce qu'**il s'agit, pour les acides inorganiques et les acides carboxyliques organiques et leurs dérivés, d'acides monocarboxyliques aliphatiques saturés et monoinsaturés ou polyinsaturés et d'acides dicarboxyliques et polycarboxyliques aliphatiques saturés et monoinsaturés ou polyinsaturés et, pour leurs dérivés, d'acides hydroxamiques, d'acides hydroxycarboxyliques, d'aldéhyde-acides et de cétoacides.

13. Procédé selon la revendication 12, **caractérisé en ce qu'**il s'agit, pour les acides protoniques, de ceux de la série acide chlorhydrique, acide sulfurique, acide sulfureux, acide nitrique, acide nitreux, acide hypochloreux, acide chloreux, acide chlorique, acide perchlorique, acides sulfoniques, acide formique, acide acétique, acide propionique, acide butyrique, acide valérianique, acide caproïque, acide heptanoïque, acides gras à plus longue chaîne, saturés et insaturés, acide oxalique, acide malonique, acide succinique, acide maléique, acide glutarique, acide citrique, acide tartrique, acide malique, acide lactique ou acide ascorbique ou leurs dérivés.

14. Procédé selon au moins l'une quelconque des revendications 12 ou 13, **caractérisé en ce qu'**on distille à une pression de 5 à 100 mbar et une température entre 30 et 120°C.
